Europäisches Patentamt

European Patent Office

Office européen des brevets

(11) Publication number: **0 274 499 B1**

# EUROPEAN PATENT SPECIFICATION

(45) Date of publication of patent specification: **17.06.92**  (51) Int. Cl.⁵: **A61B 5/042**, A61N 1/05

(21) Application number: **87904406.3**

(22) Date of filing: **03.07.87**

(86) International application number:
**PCT/IT87/00068**

(87) International publication number:
**WO 88/00449 (28.01.88 88/03)**

(54) OESOPHAGEAL PROBE PROVIDED WITH MEASURING OR STIMULATING ELECTRODES.

(30) Priority: **11.07.86 IT 346286**

(43) Date of publication of application:
**20.07.88 Bulletin  88/29**

(45) Publication of the grant of the patent:
**17.06.92 Bulletin  92/25**

(84) Designated Contracting States:
**AT DE FR SE**

(56) References cited:
**WO-A-79/00811
WO-A-81/03428
DE-A- 2 043 105
FR-A-22 376 48
US-A- 3 933 612**

(73) Proprietor: **C.B. BIOELETTRONICA s.r.l.
Via Paolo Costoli, 4
I-50039 Vicchio (FI)(IT)**

(72) Inventor: **GRASSI, Gino
Via Imbriani, 21
I-50019 Sesto Fiorentino(IT)**
Inventor: **MARCONI, Paolo
Via Mercati, 6
I-50139 Firenze(IT)**

(74) Representative: **Lanzoni, Luciano
c/o BUGNION S.p.A. Via dei Mille, 19
I-40121 Bologna(IT)**

EP 0 274 499 B1

# Description

The invention relates to a dipole probe designed for transoesophageal recording and/or stimulation of the heart, that can be inserted through the mouth of the patient.

It has been found advantageous, effecting electrophysiological tests (invasive included) in which electrical cardiac activity is monitored, to make use of electrodes positioned in the oesophagus for the purpose of recording atrial electrical activity and/or stimulating the left atrium by exploiting the close anatomical proximity of oesophagus and heart. A strong requirement exists with many heart diseases for pin-pointing atrial and ventricular signals as and when they occur, momentarily and over extended periods alike; in many instances, such signals can not be picked up and interpreted by way of normal ECG monitoring.

Utilized in their most notable applications, the types of probe in question permit of:

- distinguishing between ventricular and super-ventricular genesis of hyperkinetic arrhythmia where a wide QRS is registered;
- distinguishing between ectopic atrial heartbeat and sinoatrial stoppage;
- monitoring ventricular-auricular conduction in patients provided with pacemakers;
- monitoring the temporal relationships between QRS and P in cases of superventricular reciprocating tachyarrhythmia;
- producing stimulation, induction and/or arrest of reciprocating tachycardia.

As many arrhythmic diseases are episodic and/or paroxysmal, the facility of effecting a recording by the transoesophageal route during dynamic monitoring (Holter) for continuous periods of up to 24 hours, can prove indispensable for a correct diagnosis. Furthermore, the method in question is not invasive, and can be employed on the great number of patients without causing undue discomfort.

There is relatively little literature available on the performance of electrodes in transoesophageal probes over long periods of time.

In essence, the probes in current use, as illustrated in the publication DE-A-2043105, consist in two electrodes mechanically interconnected by means of a retaining medium constituting the body of the probe; the electrical connection is effected by way of two wires issuing from the probe, likewise axially, and passing through the patient's mouth. Damage or discomfort that could arise during the act of swallowing is avoided by enveloping the probe in a soluble capsule which, on arrival in or near the stomach, duly dissolves and frees the electrodes. Certain drawbacks remain nonetheless, amongst which are the size and shape of the capsule, often such as to make swallowing difficult for some patients and almost impossible for others.

Another drawback encountered is that the electrodes of such dipole probes exhibit somewhat pronounced corners which, when the probe is withdrawn following use, can damage the oesophageal mucous membrane.

A further drawback occurs when the capsule fails to arrive fully in the stomach, in which instance some considerable time can lapse before it dissolves in the oesophagus, and a lengthy wait is necessary before the electrophysiological tests can begin.

Yet another drawback betrayed by conventional dipole probes is that of the limited distance between the electrodes, which signifies operating with high stimulation thresholds.

Accordingly, the object of the invention is that of overcoming the drawbacks mentioned above.

The stated object is achieved with a dipole probe as described herein and as characterized in the claims appended, the electrodes of which present rounded outer surfaces and are held close together, almost to the point of making contact, by a retaining medium fashioned in gastrically soluble material.

A first advantage of the probe disclosed consists essentially in the fact that it is easily swallowed and withdrawn, as the electrodes have rounded outer surfaces, devoid of projecting edges, corners etc., and exhibit compact dimensions.

Another advantage of the probe disclosed consists in a complete absence of discomfort, which is known to be obtainable in electrodes of the type embodied without an external coating of gastrically soluble material.

Similarly advantageous is a reduction obtained in the time-lapse preceding activation of the probe, i.e. the interval that passes before the electrodes are freed and fully operational, either by rendering the two electrodes mechanically independent of one another such that their weight hastens the break-up of the retaining medium, or by interconnecting them with a spring the pent-up force of which similarly produces a destructive effect on the medium.

A further advantage of the invention is that of its greater effectiveness, achieved by adoption of a rounded shape that ensures optimum contact with the oesophagus walls.

Yet another advantage of the invention is the low stimulation threshold obtained, notwithstanding the longitudinal dimensions of the packaged probe are markedly compact. This is achieved by ensuring that the two electrodes are held close together during ingestion by no other constraint than the soluble retaining medium; the only additional connection envisaged is a spring, which in any event expands once the probe has been swallowed and the medium dissolved.

The invention will now be described in detail, by way of example, with the aid of the accompanying drawings, in which:

figs 1 and 2 show a first embodiment of the dipole probe disclosed, in axial section, viewed before and after ingestion, respectively;

figs 3 and 4 show a second embodiment of the dipole probe disclosed, in axial section, viewed before and after ingestion, respectively;

figs 5 and 6 show a further embodiment of the dipole probe disclosed, viewed in axial section and side elevation and viewed before and after ingestion, respectively.

With reference to the drawings, the orally ingested dipole electrode probe for transoesophageal cardiac recording and/or stimulation consists substantially in two electrodes 1 and 2 connected one to the other by a retaining medium, denoted 3. Within the context of the specification, "dipole" is taken to signify two electric poles, even though the number of single elements making up the electrodes may be more than two, according to the recording and/or stimulation requirements encountered. Reference is thus made to two electrodes throughout the specification, in the interests of simplicity.

One of the two electrodes 1 or 2, for instance that denoted 2, as in the drawings, is provided with an axial bore 8 affording passage to two signal leads 4 and 5 that connect with the respective electrodes 1 and 2, the lead denoted 4 also passing through the retaining medium 3 in order to reach the relative electrode 1.

According to the invention, the electrodes 1 and 2 are embodied with a rounded outer surfaces in order that no sharp corners or points will be presented.

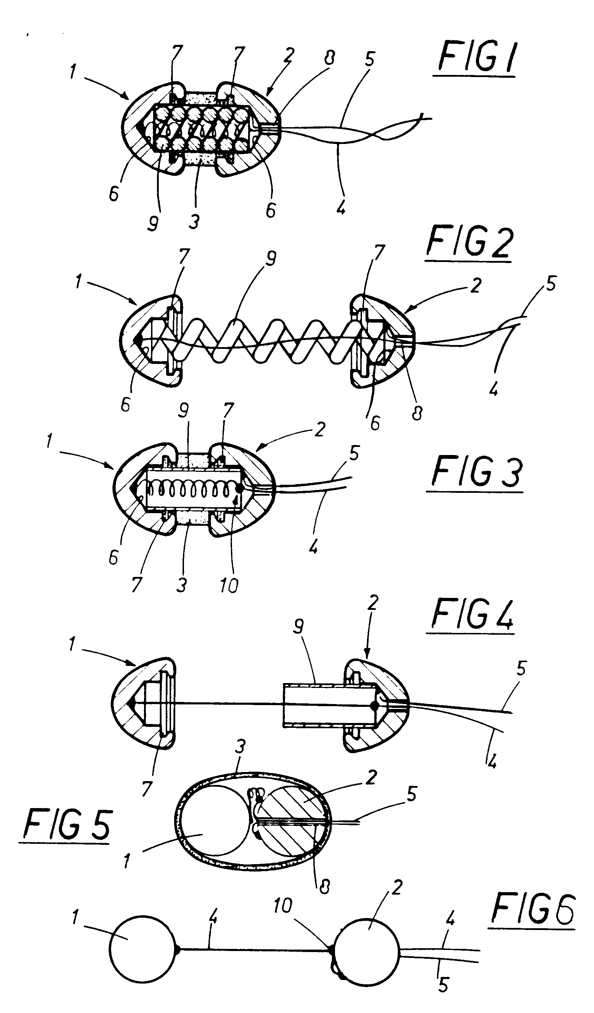

The external profile of the electrodes 1 and 2 might be spherical (as in figs 5 and 6) or elliptical (as in figs 1...4); other profiles could equally well be adopted, e.g. lenticular.

In figs 1...4, the electrodes 1 and 2 are dished, the inside of each one affording a circumferential seat 7 in which to anchor the retaining medium 3. The retaining medium is fashioned in a gastrically soluble gelatin, and proportioned with a maximum transverse dimension smaller than the corresponding dimension exhibited by each electrode 1 and 2.

The gelatin retaining medium is prevented from filling the space encompassed by the electrodes 1 and 2, to no useful purpose, by association of a further component with the internal surface 6 of at least one electrode, and more precisely, a tubular element 9 fashioned in biocompatible material such as Teflon, Silastic, Derlin or the like, which would be welded or glued in position.

In a first preferred embodiment (see figs 1 and 2), the tubular element 9 takes the form of a tightly compressed coil spring which is held in its bunched position by the retaining medium 3 until ingestion. Each end of the spring 9 is anchored to the relative electrode 1 and 2 by welding, or using an adhesive or other similar expedient. Thus, once the probe has been swallowed and passed through to the stomach, the spring 9 will hasten break-up of the retaining medium 3; the result is that the dipole probe can reach the oesophagus or the stomach and be rendered operational (see fig 2) in a much shorter time than is the case with conventional embodiments, where one has to wait for a complete break-up of the soluble gelatin to come about without any auxiliary impetus being applied.

Needless to say, the spring 9 will be fashioned in a biocompatible material such as those aforementioned. In the second embodiment illustrated (figs 3 and 4), the tubular element 9 is a simple tube fashioned in a material that is biocompatible, and soft. In this embodiment, the tubular element 9 is anchored to one only of the electrodes 1 or 2, say, the electrode 2 through which the signal leads 4 and 5 are routed; following ingestion, in fact, this electrode will be positioned uppermost, and the unattached end of the tubular element 9 will become the trailing end when the probe is withdrawn following the test.

In this embodiment, the signal lead 4 connected to the electrode 1 without the bore is provided with a stop 10 consisting, quite simply, in a knot tied in the lead 4 itself at a point either between the two electrodes 1 and 2 or externally of the probe. With the knot 10 tied between the electrodes, it will be the lead 4 of the farthest electrode 1 that must be pulled to withdraw the probe (see fig 4); with the knot 10 tied externally of the probe, the lead 5 of the nearest electrode 2 will be pulled to accomplish withdrawal.

The length of the extended spring 9, in the first embodiment, or the distance separating the farthest electrode 1 and the knot 10 in the second, will be dependent upon the spacing of the electrodes 1 and 2 required in operation.

In both of the embodiments described thus far, the drawbacks besetting conventional dipole probes are overcome -viz,

- in either embodiment, the overall dimensions of the probe obtainable for the purposes of ingestion are those of an ordinary pill, and in the second, the two components withdrawn are even smaller;

- the outer surfaces of the probe remain absolutely smooth during ingestion and withdrawal alike;

- the interval which passes before the electrodes are rendered operational is notably brief, as the force of the spring 9 in the first embodiment, or the weight of the farthest electrode 1 in the second, will have the effect of assisting break-up of the gastrically soluble gelatin retaining medium 3. In addition, the distance separating the electrodes can be made relatively great, thereby lowering the stimulation threshold; thus, notwithstanding its compact dimensions at the moment of ingestion, the dipole probe is rendered singularly effective not only for the reception of cardiac signals, but also for pacemaking purposes.

In a further embodiment illustrated in figs 5 and 6, the electrodes 1 and 2 are embodied as two balls, the one entirely solid, the other provided with an axial bore 8 affording passage to the two signal leads 4 and 5. The electrodes are held together for the purposes of ingestion by an enveloping retaining medium 3, namely a gastrically soluble capsule. Likewise in this instance, break-up of the retaining medium 3 is hastened by the weight of the farthest electrode 1, the lead 4 from which is provided with a stop 10 identical or similar to that illustrated in figs 3 and 4. The embodiment of the electrodes 1 and 2, especially small and totally devoid of any projecting edges or corners, facilitates withdrawal still further.

In order to improve signal reception, or step up the transmission capacity for pacemaking purposes, the electrodes 1 and 2 might be faced externally with a layer of precious metal, or activated by coating with platinum black.

Clear reception of cardiac signals, i.e. undisturbed by the effects of breathing and muscular activity, can be ensured by routing the signal leads 4 and 5 through a filter circuit capable of eliminating such unwanted interference.

**Claims**

1. An orally ingestable dipole probe for transoesophageal cardiac recording and/or stimulation, comprising at least two conductive elements fashioned from a biocompatible material and constituting electrodes (1, 2) which, exhibit rounded outer surfaces totally devoid of sharp edges and corners a retaining medium (3) by which the electrodes are interconnected mechanically, and at least one pair of signal leads (4, 5) wired to the electrodes, which are routed through an access bore (8),
characterized in that said bore (8) is formed in at least one electrode, and in that the electrodes (1, 2) are held close together prior to their ingestion by the retaining medium (3), which is fashioned in gastrically soluble material.

2. A dipole probe as in claim 1, wherein the electrodes (1, 2) are two in number and exhibit a dished shape affording an internal circumferential seat (7) in which to anchor a retaining medium (3) fashioned in gastrically soluble gelatin, and wherein the soluble retaining medium ensheaths a tubular element (9) fashioned in a biocompatible material and associated with the internal surface (6) of at least one of the two electrodes (1, 2).

3. A dipole probe as in claim 2, wherein the retaining medium (3) is proportioned with a maximum external diameter smaller than the maximum external diameter exhibited by the electrodes (1, 2).

4. A dipole probe as in claim 2, wherein the external profile of the single electrode (1, 2) is spherical, at least in part, or substantially elliptical or lenticular.

5. A dipole probe as in claim 2, wherein the tubular element (9) is made fast at each end to the internal surface (6) of both electrodes (1, 2) and embodied as a helical spring that is compressed into tubular configuration, its single coils urged into mutual contact one with the next, through the agency of the retaining medium (3) by which it is ensheathed.

6. A dipole probe as in claim 2, comprising a tubular element (9) fashioned from a soft material and made fast at one end to the internal surface (6) of one electrode (1, 2) only, wherein the signal lead (4) wired to the electrode (1) other than that through which the leads (4, 5) are routed is provided with a stop (10) serving to dictate the distance by which the two electrodes (1, 2) will be separated once the retaining medium (3) has been dissolved, or at least broken up, and the lead (4) itself fully extended.

7. A dipole probe as in claim 6, wherein the tubular element (9) is made fast by one end to the electrode (2) through which the signal leads (4, 5) are routed.

8. A dipole probe as in claim 6, wherein the tubular element (9) is made fast by one end to the electrode (1) other than that through which the signal leads (4, 5) are routed.

9. A dipole probe as in claim 1, wherein the electrodes (1, 2) are ball shaped and enveloped by a retaining medium (3) embodied as a capsule of gastrically soluble material, and wherein the signal lead (4) wired to the electrode (1) other than that through which the leads (4, 5) are routed is provided with a stop (10) serving to dictate the distance by which the two electrodes (1, 2) will be separated once the retaining medium (3) has been dissolved, or at least broken up, and the lead (4) itself fully extended.

10. A dipole probe as in claim 6 or 9, wherein the stop (10) consists in a knot tied in the signal lead (4) wired to the electrode (1) other than that through which the leads (4, 5) are routed, at a given point between the electrodes (1, 2).

11. A dipole probe as in claim 6 or 9, wherein the stop (10) consists in a knot tied in the signal lead (4) wired to the electrode (1) other than that through which the leads (4, 5) are routed, at a given point other than between the electrodes (1, 2).

12. A dipole probe as in claim 1, wherein the outer surface of the electrodes (1, 2) is either faced with a layer of precious metal, or activated.

13. A dipole probe as in claim 1, wherein the outer surface of the electrodes (1, 2) is activated by application of a coating of platinum black.

**Revendications**

1. Sonde dipôle à ingérer à travers la bouche pour la mesure et/ou la stimulation oesophagienne du coeur, comportant au moins deux éléments conducteurs produits en matière biocompatible et constituant les électrodes (1, 2) lesquelles présentent des surfaces extérieures arrondies complètement dépourvues de bords saillants et arêtes vives, un moyen de retenue (3) par lequel les électrodes sont reliées l'une à l'autre mécaniquement, et au moins deux fils conducteurs (4, 5) de signalisation connectés aux électrodes, lesquels sont acheminés à travers un trou d'accès (8), caractérisée en ce que ledit trou (8) est ménagé dans au moins une électrode et en ce que les électrodes (1, 2) sont maintenues rapprochées l'une de l'autre avant leur ingestion par le moyen de retenue (3) qui est réalisé en matière gastrosoluble.

2. Sonde dipôle selon la revendication 1, caractérisée en ce que les électrodes (1, 2) sont au nombre de deux et présentent une forme en cuvette pourvue d'un logement intérieur circonférentiel (7) dans lequel est ancré un moyen de retenue (3) fabriqué en gélatine gastrosoluble, et en ce que le moyen de retenue soluble enveloppe un élément tubulaire (9) de matière biocompatible et associé à la surface intérieure (6) de l'une au moins des deux électrodes (1, 2).

3. Sonde dipôle selon la revendication 2, caractérisée en ce que le moyen de retenue (3) a un diamètre extérieur maximum plus petit que le diamètre extérieur maximum présenté par les électrodes (1, 2).

4. Sonde dipôle selon la revendication 2, caractérisée en ce que le profil extérieur de chaque électrode individuelle (1, 2) est sphérique, au moins partiellement, ou sensiblement elliptique ou lenticulaire.

5. Sonde dipôle selon la revendication 2, caractérisée en ce que l'élément tubulaire (9) est fixé par ses extrémités opposées à la surface intérieure (6) des deux électrodes (1, 2) et est concrétisé par un ressort en spirale comprimé de manière à prendre une configuration tubulaire, les spires étant poussées les unes au contact des autres à l'aide du moyen de retenue (3) par lequel elles sont entourées.

6. Sonde dipôle selon la revendication 2, comprenant un élément tubulaire (9) obtenu d'une matière souple et moelleuse et fixé par l'une de ses extrémités à la surface intérieure (6) de l'une des électrodes (1, 2) seulement, caractérisée en ce que le fil conducteur (4) de signalisation connecté à l'électrode (1) qui n'est pas celle à travers laquelle passent les fils conducteurs (4, 5) est pourvu d'un arrêt (10) servant à établir la distance par laquelle les deux électrodes (1, 2) sont séparées quand le moyen de retenue (3) a été dissous ou au moins brisé, et le fil (4) est complètement étendu.

7. Sonde dipôle selon la revendicationn 6, caractérisée en ce que l'élément tubulaire (9) est fixé par l'une de ses extrémités à l'électrode (2) à travers laquelle passent les fils conducteurs (4, 5) de signalisation.

8. Sonde dipôle selon la revendication 6, caractérisée en ce que l'élément tubulaire (9) est fixé par l'une de ses extrémités à l'électrode (1) différente de celle à travers laquelle passent les fils conducteurs (4, 5) de signalisation.

9. Sonde dipôle selon la revendication 1, caractérisée en ce que les électrodes (1, 2) sont en forme de sphère et enveloppées par le moyen de retenue (3) concrétisé par une capsule de matière gastrosoluble, et en ce que le fil conducteur (4) de signalisation connecté à l'électrode (1) différente de celle à travers laquelle passent les fils conducteurs (4, 5) est pourvu d'un arrêt servant à établir la distance par laquelle les deux électrodes (1, 2) seront séparées quand le moyen de retenue (3) a été dissous ou au moins brisé, et le fil (4) est complètement étendu.

10. Sonde dipôle selon la revendication 6 ou 9, caractérisée en ce que l'arrêt (10) est représenté par un noeud exécuté sur le fil conducteur (4) de signalisation connecté à l'électrode (1) différente de celle à travers laquelle passent les fils (4, 5), à un endroit donné entre les électrodes (1, 2).

11. Sonde dipôle selon la revendication 6 eu 9, caractérisée en ce que l'arrêt (10) est représenté par un noeud exécuté sur le fil conducteur (4) de signalisation connecté à l'électrode (1) différente de celle à travers laquelle passent les fils (4, 5), à un endroit donné qui ne se trouve pas entre les électrodes (1, 2).

12. Sonde dipôle selon la revendication 1, caractérisée en ce que la surface extérieure des électrodes (1, 2) est revêtue d'une couche de métal précieux ou activée.

13. Sonde dipôle selon la revendication 1, caractérisée en ce que la surface extérieure des électrodes (1, 2) est activée moyennant l'application d'un revêtement de noir de platine.

**Patentansprüche**

1. Eine durch den Mund einführbare zweipolige Speiseröhrensonde mit Herzmess- oder -stimulationselektroden, enthaltend wenigstens zwei leitende Elemente, hergestellt aus einem bioverträglichen Material und Elektroden (1, 2) bildend, welche abgerundete äussere Oberflächen aufweisen, also vollkommen frei von scharfen Ecken und Kanten; ein Haltemittel (3), durch das die Elektroden mechanisch miteinander verbunden sind; und wenigstens ein Paar von an die Elektroden angeschlossenen Signalleitern (4, 5), die durch eine Zugangsbohrung (8) geführt werden,
dadurch gekennzeichnet, dass die genannte Bohrung (8) in wenigstens eine Elektrode eingearbeitet ist, und dass die Elektroden (1, 2) vor ihrem Einführen durch das Haltmittel (3), das aus einem gastrisch lösbaren Material hergestellt ist, dicht zusammengehalten werden.

2. Zweipolige Sonde nach Patentanspruch 1, dadurch gekennzeichnet, dass die Elektroden (1, 2) zwei in der Zahl sind und eine schalenartige Form aufweisen, und die einen internen umlaufenden Sitz (7) bieten, in welchem ein Haltemittel (3) aus gastrisch lösbarer Gelatine verankert wird, und dadurch, dass das lösbare Haltemittel ein rohrförmiges Element (9) umschliesst, das aus einem bioverträglichen Material hergestellt und mit der internen Oberfläche (6) von wenigstens einer der beiden Elektroden (1, 2) verbunden ist.

3. Zweipolige Sonde nach Patentanspruch 2, dadurch gekennzeichnet, dass das Haltemittel (3) mit einem maximalen Aussendurchmesser ausgelegt ist, der geringer ist als der maximale Aussendurchmesser der Elektroden (1, 2).

4. Zweipolige Sonde nach Patentanspruch 2, dadurch gekennzeichnet, dass die äussere Form der einzelnen Elektrode wenigstens zu einem Teil kugelförmig ist oder im wesentlichen ellipsen- oder linsenförmig.

5. Zweipolige Sonde nach Patentanspruch 2, dadurch gekennzeichnet, dass das rohrförmige Element (9) mit jedem Ende an der internen Oberfläche (6) der beiden Elektroden (1, 2) befestigt ist und aus einer Schneckenfeder besteht, die rohrförmig zusammengedrückt gehalten wird, und deren einzelne Windungen in gegenseitigem Kontakt miteinander liegen, und zwar durch die Wirkung des Haltemittels (3), durch welches sie umschlossen wird.

6. Zweipolige Sonde nach Patentanspruch 2, enthaltend ein rohrförmiges Element (9), hergestellt aus einem weichen Material und mit einem Ende an der internen Oberfläche (6) von nur einer Elektrode (1, 2) befestigt, dadurch gekennzeichnet, dass der Signalleiter (4), der an die Elektrode (1) angeschlossen wird, durch die nicht die Leiter (4, 5) geführt werden, mit einem Stopper (10) versehen ist, der zur Bestimmung des Abstandes dient, welchen die beiden Elektroden (1, 2) voneinander haben sollen, sobald sich das Haltemittel (3) aufgelöst hat oder wenigstens aufgebrochen ist und der Leiter (4) sich vollständig ausgedehnt hat.

7. Zweipolige Sonde nach Patentanspruch 6, dadurch gekennzeichnet, dass das rohrförmige Element (9) mit einem Ende an der Elektrode (2) befestigt ist, durch welche die Signalleiter (4, 5) geführt werden.

8. Zweipolige Sonde nach Patentanspruch 6, dadurch gekennzeichnet, dass das rohrförmige Element (9) mit einem Ende an der Elektrode (1) befestigt ist, durch welche nicht die Signalleiter (4, 5) geführt werden.

9. Zweipolige Sonde nach Patentanspruch 1, dadurch gekennzeichnet, dass die Elektroden (1, 2) ballförmig ausgebildet sind und von einem Haltemittel (3) umschlossen werden, das ala eine Kapsel aus gastrisch lösbarem Material ausgelegt ist, und dadurch, dass der an die Elektrode (1), durch die nicht die Leiter (4, 5) geführt werden, angeschlossene Leiter (4), mit einem Stopper (10) versehen ist, der dazu dient, den Abstand zu bestimmen, durch den die beiden Elektroden (1, 2) getrennt werden, wenn das Haltemittel (3) aufgelöst oder wenigstens aufgebrochen ist und der Leiter (4) selbst sich vollkommen gedehnt hat.

10. Zeipolige Sonde nach Patentanspruch 6 oder 9, dadurch gekennzeichnet, dass der Stopper (10) aus einem Knoten in dem Signalleiter (4) besteht, der an die Elektrode (1) angeschlossen ist, durch die nicht die Leiter (4, 5) geführt werden, und zwar an einem bestimmten Punkt zwischen den Elektroden (1, 2).

11. Zweipolige Sonde nach Patentanspruch 6 oder 9, dadurch gekennzeichnet, dass der Stopper (10) aus einem Knoten in dem Signalleiter (4) besteht, der an die Elektrode (1) angeschlossen ist, durch die nicht die Leiter (4, 5) geführt werden, und zwar an einem bestimmten Punkt, der nicht zwischen den beiden Elektroden (1, 2) liegt.

12. Zweipolige Sonde nach Patentanspruch 1, dadurch gekennzeichnet, dass die äussere Oberfläche der Elektroden (1, 2) mit einer Schicht aus Edelmetall versehen oder aktiviert ist.

13. Zweipolige Sonde nach Patentanspruch 1, dadurch gekennzeichnet, dass die äussere Oberfläche der Elektroden (1, 2) durch das Aufbringen einer Schicht aus Platinschwarz aktiviert ist.

FIG 1

FIG 2

FIG 3

FIG 4

FIG 5

FIG 6